(19) Europäisches Patentamt
European Patent Office
Office européen des brevets

(11) Publication number: **0 207 728 A2**

(12) **EUROPEAN PATENT APPLICATION**

(21) Application number: 86304910.2

(22) Date of filing: 25.06.86

(51) Int.Cl.⁴: C 07 D 231/06
A 01 N 43/56

(30) Priority: 27.06.85 JP 141314/85
16.04.86 JP 86061/86

(43) Date of publication of application:
07.01.87 Bulletin 87/2

(84) Designated Contracting States:
CH DE FR GB IT LI NL

(71) Applicant: MITSUI TOATSU CHEMICALS, INCORPORATED
2-5 Kasumigaseki 3-chome
Chiyoda-Ku Tokyo 100(JP)

(72) Inventor: Kitajima, Kouji
2882-3-18, Iijima-cho Totsuka-ku
Yokohama-shi Kanagawa-ken(JP)

(72) Inventor: Kodaka, Kenji
10-2, Kagetori-cho Totsuka-ku
Yokohama-shi Kanagawa-ken(JP)

(72) Inventor: Numata, Satoshi
1541, Yabe-cho Totsuka-ku
Yokohama-shi Kanagawa-ken(JP)

(72) Inventor: Ooka, Masayuki
4-1-28, Hase
Kamakura-shi Kanagawa-ken(JP)

(72) Inventor: Fukushi, Yukiharu
2070, Iijima-cho Totsuka-ku
Yokohama-shi Kanagawa-ken(JP)

(72) Inventor: Udagawa, Takatoshi
93-6, Tsutsumi
Chigasaki-shi Kanagawa-ken(JP)

(72) Inventor: Shiraishi, Shiro
1541, Yabe-cho Totsuka-ku
Yokohama-shi Kanagawa-ken(JP)

(72) Inventor: Nakamura, Masahiko
2282-4-37, Iijima-cho Totsuka-ku
Yokohama-shi Kanagawa-ken(JP)

(74) Representative: Stuart, Ian Alexander et al,
MEWBURN ELLIS & CO. 2/3 Cursitor Street
London EC4A 1BQ(GB)

(54) **Novel pyrazoline compounds, process for production thereof, and insecticides containing them as active ingredients.**

(57) Novel pyrazoline compounds of the general formula

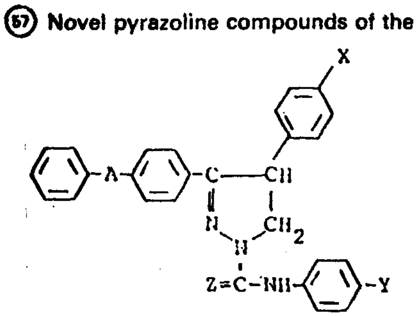

(I)

wherein X represents a hydrogen or halogen atom or a methyl or methylsulfonyloxy group, Y represents a hydrogen or halogen atom or a trifluoromethyl group, Z represents an oxygen or sulfur atom, and A represents an oxygen atom or –NH–, which are useful as an insecticide. These compounds are produced by reacting a compound represented by the general formula

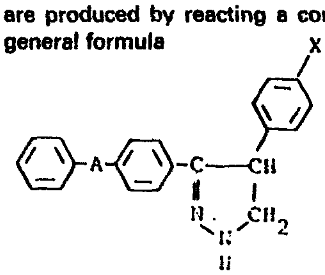

(II)

EP 0 207 728 A2

0207728

# NOVEL PYRAZOLINE COMPOUNDS, PROCESS FOR PRODUCTION THEREOF, AND INSECTICIDES CONTAINING THEM AS ACTIVE INGREDIENTS

This invention relates to a pyrazoline compound represented by the general formula

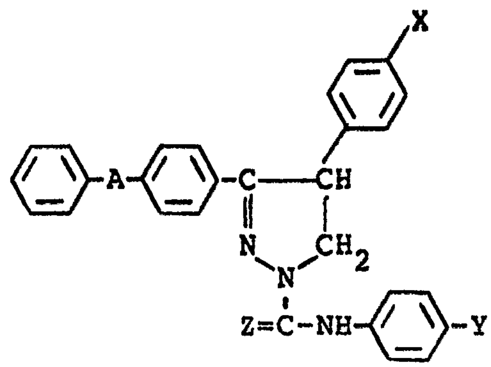

(I)

wherein X represents a hydrogen or halogen atom or a methyl or methylsulfonyloxy group, Y represents a hydrogen or halogen atom or a trifluoromethyl group, Z represents an oxygen or sulfur atom, and A represents an oxygen atom or -NH-,

a process for its production, and to an insecticide comprising it as an active ingredient.

This compound is useful in various industrial fields, especially in the agricultural field as an insecticide.

Research and development work on insecticides have been done over many years, and have resulted in the development and practical acceptance of numerous chemicals, for example organochlorine insecticides such as DDT and BHC, organophosphorous insecticides such as parathion and malathion, and carbamate insecticides such as carbaryl and methomyl. Use of some of these insecticides

has been restricted, however, because they give rise to problems of persistence, accumulation and environmental pollution. Furthermore, some insect pests have acquired resistance to these insecticides as a result of using them over many years. On the other hand, pyrethroid insecticides have been developed and gained practical acceptance which have low mammalian toxicity and easily decompose in a natural environment with little persistence and whose decomposition products are non-toxic. However, insect pests having resistance to some of these pyrethroid insecticides have recently appeared. It is desired therefore to develop a novel chemical having outstanding insecticidal property.

Embodiments of the invention may allow one to solve the aforesaid problems of the prior art, and to provide an excellent insecticidal compound having a new structure, a broad insecticidal spectrum and high insecticidal activity.

According to the invention there is provided a novel pyrazoline compound represented by the following general formula

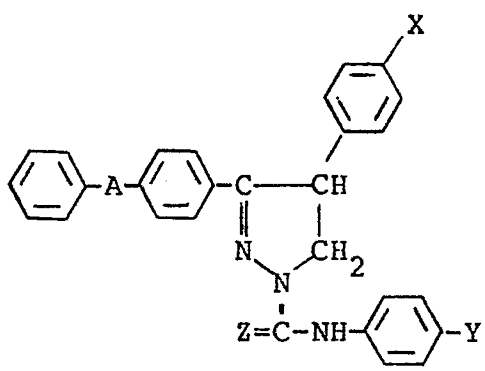

(I)

wherein X represents a hydrogen or halogen atom or a methyl or methylsulfonyloxy group, Y represents a hydrogen or halogen atom or a trifluoromethyl group, Z represents an oxygen

or sulfur atom, and A represents an oxygen atom or -NH-.

They are novel compounds discovered for the first time by the present inventor and are not described in the prior litetrature.

According to this invention, there is also provided a process for producing pyrazoline compound represented by the following general formula

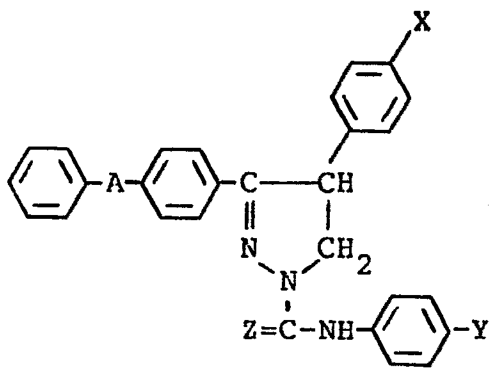

(I)

wherein X represents a hydrogen or halogen atom or a methyl or methylsulfonyloxy group, Y represents a hydrogen or halogen atom or a trifluoromethyl group, Z represents an oxygen or sulfur atom, and A represents an oxygen atom or -NH-,

which comprises reacting a compound represented by the general formula

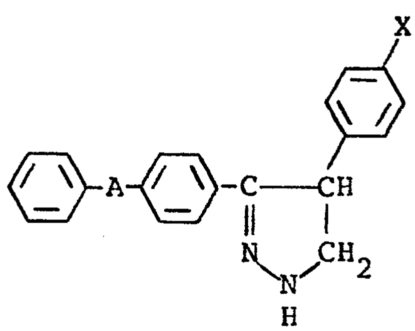

(II)

wherein X and A are as defined above,

with a compound represented by the general formula

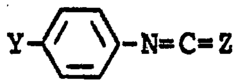

(III)

wherein Y and Z are as defined above.

According to this invention, there is also provided an insecticide comprising a pyrazoline compound represented by the above general formula (I)

DETAILED DESCRIPTION OF THE INVENTION

This invention relates to novel pyrazoline compounds of the general formula (I)

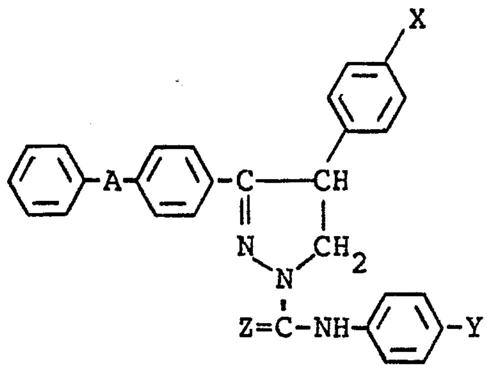

(I)

wherein X is a hydrogen or halogen atom or a lower alkyl group, especially methyl group or methylsulfonyloxy group, Y is a hydrogen or halogen atom or a trifluoromethyl group, Z is an oxygen or sulfur atom, and A represents an oxygen atom or -NH-,

which are useful as an insecticide.

The process of this invention for producing the compound of general formula (I) is schematically shown as follows.

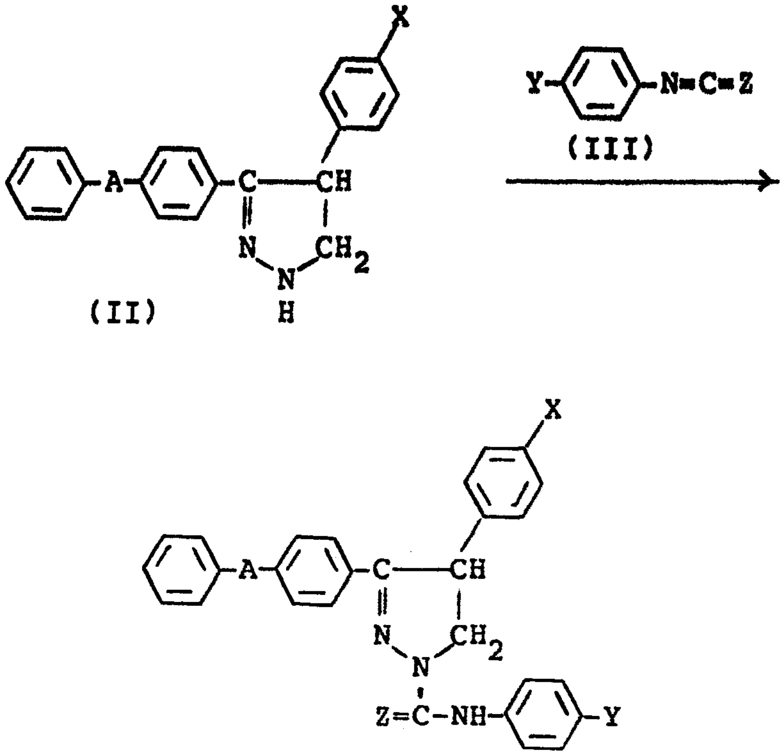


(II)

(I)

(X, Y, Z and A are as defined above.)

Specifically, a 3-aryl-4-phenyl-2-pyrazoline derivative represented by general formula (II) is reacted with a phenyl isocyanate or isothiocyanate derivative represented by general formula (III) in the presence or absence of an inert solvent. Examples of the inert solvent are tetrahydrofuran, ethyl ether, benzene, toluene, acetonitrile, pyridine, dichloromethane, chloroform, carbon tetrachloride and 1,3-dimethyl-2-imidazolidinone.

The reaction temperature and time may be varied widely according to the starting compounds. Generally, the reaction temperature is $-10^{o}C$ to $+120^{o}C$, preferably $0^{o}C$ to $100^{o}C$, and the reaction time is generally 0.1 to 30 hours, preferably 0.5 to 24 hours.

A starting compound of general formula (II) in which A is an oxygen atom [compound of formula (IIa)] can be synthesized in accordance with the following scheme.

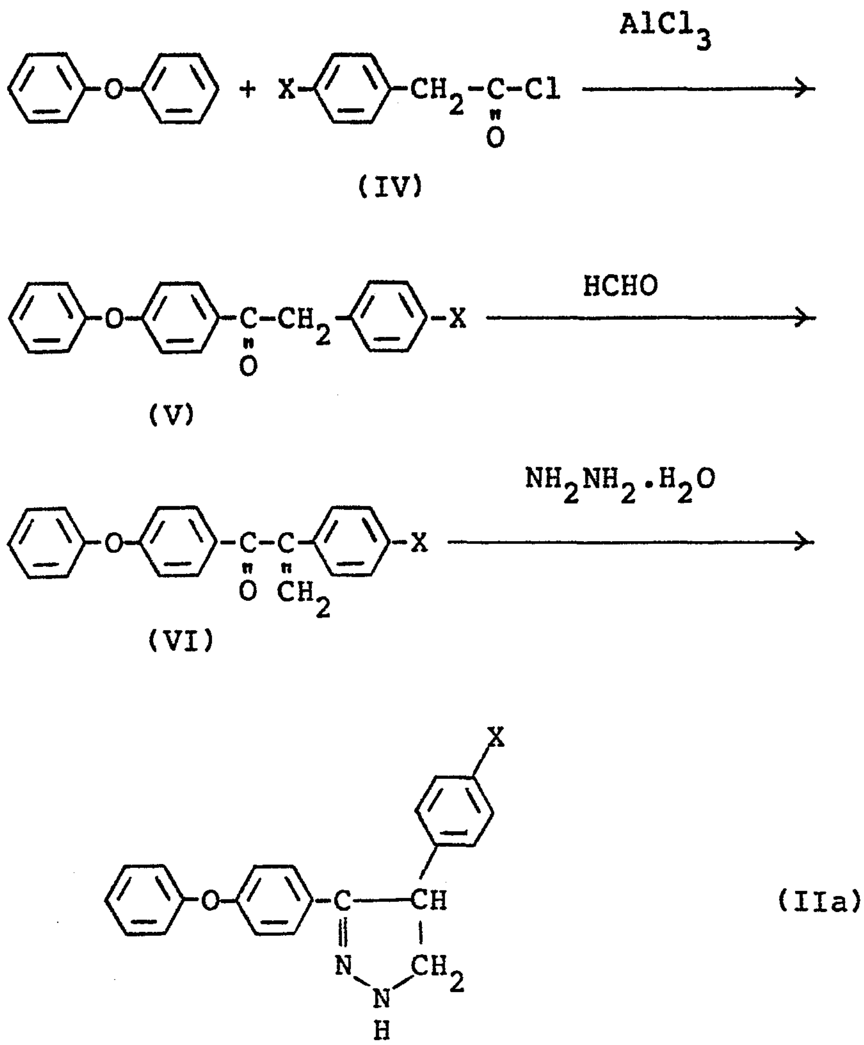


(IV)

(V)

(VI)

(IIa)

(X is as defined above.)

Specifically, the pyrazoline derivative of general formula (IIa) can be produced by reacting diphenyl ether with a phenyl acetyl chloride of general formula (IV), for example in the presence of anhydrous aluminum chloride, reacting the resulting 4-phenoxyphenyl benzyl ketone derivative of general formula (V) with formaldehyde in the presence of a catalyst in a solvent such as methanol or ethanol, and reacting the resulting compound of general formual (VI) with hydrazine in an alcohol solvent such as methanol, ethanol or isopropanol.

A starting compound of general formula (II) in which A is -NH- [compound of general formula (IIb)] can be synthesized in accordance with the following scheme.

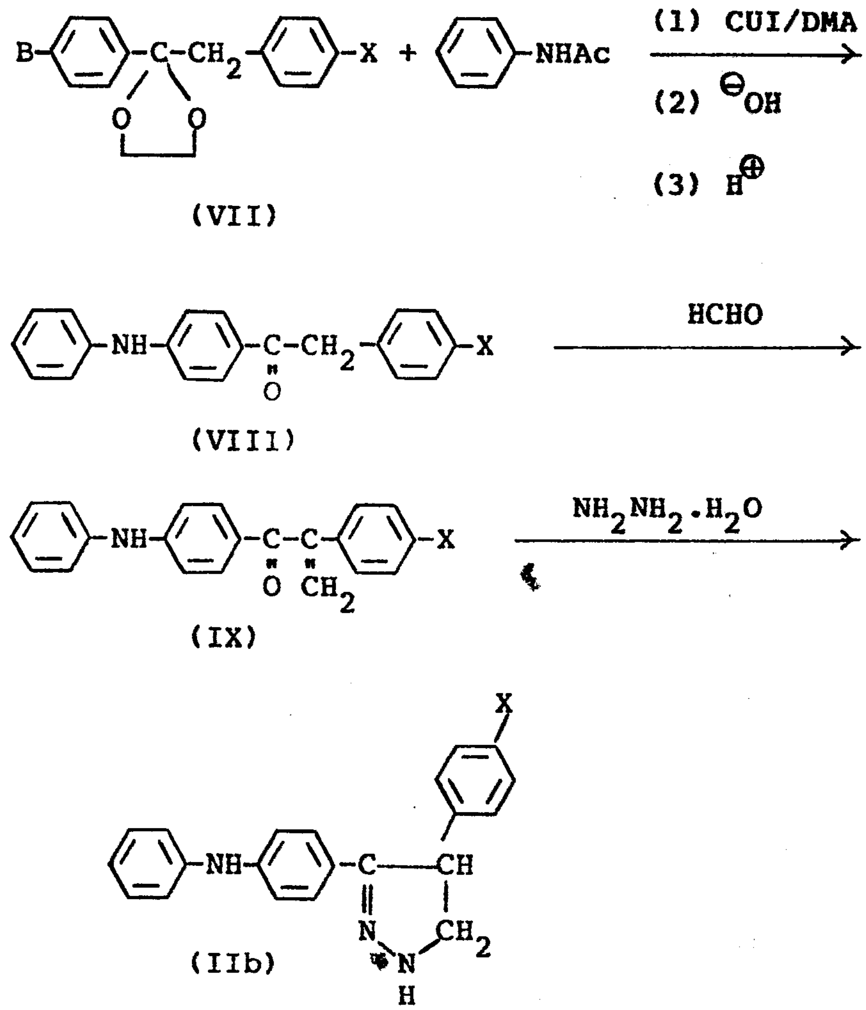


(In the formulae, X is as defined above.)

Specifically, the pyrazoline derivative of general formula (IIb) can be produced by reacting acetanilide with an ethylene acetal derivative of general formula (VII) in dimethylacetamide solvent, for example, in the presence of cuprous iodide, thereafter treating the product with an alkali and an acid, reacting the resulting benzylketone derivative of formula (VIII) with formaldehyde in the presence of a catalyst in a solvent such as methanol or ethanol, and reacting the resulting

compound of formula (IX) with hydrazine in an alcohol sovlent such as methanol, ethanol or isopropanol.

Certain 3-aryl-4-phenyl-2-pyrazoline derivatives are unstable and decompose even at room temperature, and therefore require storage in a cold and dark place in an atmopshere of nitrogen (EP 58,424). In contrast, the 3-aryl-4-phenyl-2-pyrazoline derivatives represented by general formula (II) provided by this ivnention are stable and can be handled easily at room temperature without decomposition. Hence, there is no restriction on the industrial production of these compounds.

The compounds of this invention represented by general formula (I) are effective against sanitary insect pests such as flies, mosquitoes and cockroaches; agricultural insect pests, for example hemipterous insect pests such as small brown planthopper (Laodelphax striatellus Fallén), brown planthopper (Nilaparvata lugens Stål), white-backed planthopper (Sogatella furcifera Horvath), green rice leafhopper (Nephotettix cincticeps Uhler), westwood-greenhouse whitefly (Trialeurodes vaporariorum) and green peach aphid (Myzus persicae Sulzer); lepidopterous insect pests such as apple leafminer (Phyllonorycter ringoneella Matsumura), diamondback moth (Plutella xylostella Curtis), armyworm (Pseudaletia separata Walker), cabbage armyworm (Mamestra brassicae Linné), tobacco cutworm (Spodoptera litura Fablicius) and common cabbageworm (Pieris rapae crucivora Boisduval); coleopterous insect pests such as rice leaf beetle (Oulema oryzae Kuwayama) and rice plant weevil (Echinocnemus squameus Billberg); and household insect pests such as termites and bark beetles, and show outstanding effect against the lepidopterous insect pests.

In actual application, the compounds of this invention may be used singly, but for ease of use as controlling agents, it is the general practice to mix

them with carriers. Formulating the compounds of this invention does not require special conditions. They can be formulated into any desired forms such as emulsifiable concentrates, wettable powders, dusts, granules, micro-granules, oil solutions, aerosols and poison baits by any methods known in the art in the production of general agricultural chemicals, and may be used in various modes of application.

The carriers as used herein denote syntheic or natural inorganic or orgnaic materials which are incorporated to aid the active ingredient to reach the site of treatment, or to facilitate storage, transportation and handling of the active ingredient.

Examples of suitable solid carriers include clays such as montmorillonite and kaolinite; inorganic substances such as diatomaceous earth, terra alba, talc, vermiculite, gypsum, calcium carbonate, silica gel and ammonium sulfate; organic materials of plant origin such as soybean meal, sawdust and wheat flour, and urea.

Examples of suitable liquid carriers include aromatic hydrocarbons such as toluene, xylene and cumene; paraffinic hydrocarbons such as kerosene and mineral oils; halogenated hydrocarbons such as carbon tetrachloride, chloroform and dichloroethane; ketones such as acetone and methyl ethyl ketone; ethers such as dioxane and tetrahydrofuran; alcohols such as methanol, ethanol, propanol and ethylene glycol; dimethylformamide; dimethyl sulfoxide; and water.

To enhance the efficacy of the compounds of this invention, various adjuvants may be used singly or in combination according to the type of the formulation, the locus to be applied, etc. Examples of adjuvants used for the purpose of emulsification, dispersing, spreading, wetting, binding and stabilization include water-soluble bases such as ligninsulfonic acid salts; anionic surface-active agents such as alkylbenzenesulfonic acid salts;

non-ionic surface-active agents such as alkylsulfuric acid esters; polyoxyalkylene alkyl ethers, polyoxyalkylene alkyl aryl ethers, polyoxyalkylene alkylamines, polyoxyalkylene alkylamides, polyoxyalkylene alkylthioethers, polyoxyalkylene fatty acid esters, glycerin fatty acid esters, sorbitan fatty acid esters, polyoxyalkylene sorbitan fatty acid esters and polyoxypropylene polyoxyethylene block polymers; lubricants such as calcium stearate and waxes; stabilizers such as isopropyl hydrogen phosphate; and other additives such as methyl cellulose, carboxymethyl cellulose, casein and gum arabic. These examples are not limitative.

By using two or more compounds of this invention, higher insecticidal activity may be obtained.

A multipurpose composition having a higher efficacy may be prepared by mixing the compound of this invention with another bioactive substance. The mixing is expected to produce a synergistic effect. Examples of such a bioactive substance include pyrethrum extract; synthetic pyrethroids and their isomers such as allethrin, N-(chrysanthemoylmethyl)-3,4,5,6-tetrahydrophthalimide, 5-benzyl-3-furylmethyl chrysanthemate, 3-phenoxybenzyl chrysanthemate, 5-propargylfurfuryl chrysanthemate, cyclopropanecarboxylic acid esters [such as 3-phenoxybenzyl 2,2-dimethyl-3-(2,2-dichlorovinyl)-cyclopropane-1-carboxylate, 3-phenoxy-α-cyanobenzyl 2,2-dimethyl 3-(2,2-dichlorovinyl)-cyclopropane-1-carboxylate, and 3-phenoxy-α-cyanobenzyl 2,2-dimethyl-3-(2,2-dibromovinyl)-cyclopropane-1-carboxylate)] and 3-phenoxy-α-cyanobenzyl α-isopropyl-4-chlorophenylacetate; organophosphorous insecticides such as O,O-diethyl-O-(3-oxo-2-phenyl-2H-pyridazin-6-yl) phosphorothioate (OFUNAC, a registered trademark of Mitsui Toatsu Chemical Inc.), O,O-dimethyl-O-(2,2-dichlorovinyl)phosphate (DDVP), O,O-dimethyl-O-(3-methyl-4-nitrophenyl)phosphorothioate, diazinon, O,O-dimethyl-O-4-cyanophenyl

phosphorothioate, O,O-dimethyl-S-[α-(ethoxycarbonyl)-benzyl]phosphorodithioate, 2-methoxy-4H-1,3,2-benzodioxaphospholin-2-sulfide, and O-ethyl-O-4-cyanophenyl phosphonothioate; carbamate insecticides such as 1-naphthyl N-methylcarbamate (NAC), m-tolyl N-methylcarbamate (MTMC), 2-dimethylamino-5,6-dimethylpyrimidin-4-yl-dimethylcarbamate (pyrimer), 3,4-dimethylphenyl N-methylcarbamate and 2-isopropoxyphenyl N-methylcarbamate; aryl propyl ether-type insecticides such as 3-phenoxybenzyl 2-(4-chlorophenyl)-2-methylpropyl ether, 3-phenoxy-4-fluorobenzyl 2-(4-chlorophenyl)-2-methylpropylether, 3-phenoxybenzyl 2-(4-ethoxyphenyl)-2-methylpropyl ether, and 3-phenoxy-4-fluorobenzyl 2-(4-ethoxyphenyl)-2-methylpropyl ether; aromatic alkane insecticides such as 1-(3-phenoxyphenyl)-4-(4-chlorophenyl)-4-methylpentane, 1-(3-phenoxy-4-fluorophenyl)-4-(4-chlorophenyl)-4-methylpentane, 1-(3-phenoxyphenyl)-4-(4-ethoxyphenyl)-4-methylpentane and 1-(3-phenoxy-4-fluorophenyl)-4-(4-ethoxyphenyl)-4-methylpentane; other insecticides; miticides; fungicides; nematocides; herbicides; plant growth regulators; fertilizers; BT agents; insect hormones; and other agricultural chemicals.

The compounds of general formula (I) according to the present invention are stable to light, heat, oxidation, etc. As required, however, a more stable insecticidal composition may be obtained by adding a suitable amount of an antioxidant or an ultraviolet absorber, for example phenol derivatives such as BHT (2,6-di-t-butyl-4-methylphenol) and BHA (butylhydroxyanisole), a bisphenol derivative, and an arylamine such as phenyl-α-naphthylamine, phenyl-β-naphthylamine or a condensate of phenetidine and acetone, and benzophenone derivative as a stabilizer.

The insecticide of this invention desirably contains 0.0001 to 95 % by weight, preferably 0.001 to 50% by weight, of the compound of general formula (I) as

an active ingredient. Desirably, the insecticide of this invention is applied in an active ingredient concentration of generally 0.01 to 5,000 ppm, preferaly 0.1 to 1,000 ppm. The rate of its application per 10a is generally 300 g to 1 g as the active ingredient.

Typical examples of the compounds of this invention represented by general formula (I) are shown in Table 1 without any intention of limiting the invention thereto.

Table 1

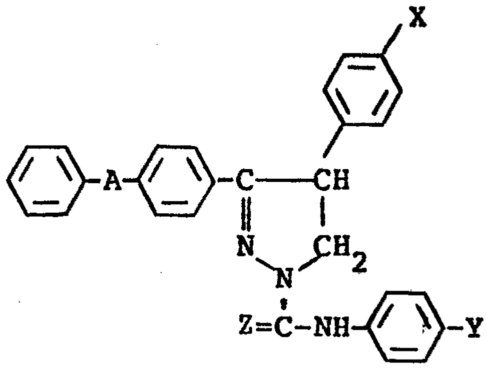

| Compound No. | A | X | Y | Z | Melting point ($^{o}C$) |
|---|---|---|---|---|---|
| 1 | 0 | H | Cl | O | 161.2 - 162.8 |
| 2 | 0 | Cl | H | O | 162.6 - 165.5 |
| 3 | 0 | Cl | Cl | O | 158.7 - 160.9 |
| 4 | 0 | Cl | F | O | 133.0 - 134.5 |
| 5 | 0 | F | H | O | 151.0 - 152.4 |
| 6 | 0 | F | Cl | O | 150.6 - 151.7 |
| 7 | 0 | F | F | O | 122.6 - 123.4 |
| 8 | 0 | $CH_3$ | F | O | 160.8 - 163.0 |
| 9 | 0 | Cl | F | S | 173.5 - 173.8 |
| 10 | 0 | F | $CF_3$ | O | 175.8 - 176.5 |
| 11 | 0 | H | F | O | 134.2 - 134.8 |
| 12 | 0 | $OSO_2CH_3$ | F | O | 168 - 171 |
| 13 | 0 | Br | Cl | O | 166.5 - 168.0 |
| 14 | 0 | Br | F | O | 146.5 - 147.5 |
| 15 | -NH- | H | Cl | O | 197.0 - 199.0 |
| 16 | -NH- | H | F | O | 188.0 - 189.3 |
| 17 | -NH- | F | F | O | 155.0 - 156.5 |
| 18 | -NH- | F | Cl | O | 145.0 - 146.5 |
| 19 | -NH- | Cl | F | O | 153.0 - 154.5 |
| 20 | -NH- | Cl | Cl | O | 145.0 - 146.0 |

The following Synthesis Examples specifically illustrate the production of the compounds of this invention.

SYNTHESIS EXAMPLE 1

Synthesis of 1-(4-chlorophenylcarbamoyl)-3-(4-phenoxyphenyl)-4-phenyl-2-pyrazoline (compound No. 1):-

In 50 ml of anhydrous tetrahydrofuran was dissolved 7.8g of 4-chlorophenyl isocyanate, and 16.0 g of 3-(4-phenoxyphenyl)-4-phenyl-2-pyrazoline was added. The mixture was stirred at room temperature for 6 hours. The solvent was evaporated under reduced pressure to give 23.8g of an oily residue. The oily residue was crystallized from ethanol to give 20.4g of pure 1-(4-chlorophenylcarbamoyl)-3-(4-phenoxyphenyl)-4-phenyl-2-pyrazoline.

Melting point: 161.2 - 162.8 $^{o}C$

$\nu_{max}^{KBr}$ $(cm^{-1})$: 3200, 1645, 1580, 1520, 1480, 1420, 1400, 1230.

$\delta_{TMS}^{CDCl_3}$ (ppm): 4.00 (1H, dd, J=5Hz, 11Hz), 4.33 (1H, t, J=11Hz), 4.66 (1H, dd, J=5Hz, 11Hz), 6.7-7.7 (18H, m), 8.08 (1H, broad s).

Elemental analysis values: $C_{28}H_{22}ClN_3O_2$

| | C | H | Cl | N |
|---|---|---|---|---|
| Calculated (%) | 71.86 | 4.74 | 7.58 | 8.98 |
| Found (%) | 71.74 | 4.78 | 7.49 | 8.85 |

SYNTHESIS EXAMPLE 2

Synthesis of 1-(4-chlorophenylcarbamoyl)-3-(4-phenoxyphenyl)-4-(4-chlorophenyl)-2-pyrazoline (compound No. 3):-

In 50 ml of anhydrous 1,3-dimethyl-2-imidazolidinone was dissolved 8.0g of 4-chlorophenyl isocyanate, and 18.0g of 3-(4-phenoxyphenyl)-4-(4-chlorophenyl)-2-pyrazoline was added. The mixture was stirred at room

temperature for 3 hours. The reaction mixture was poured into water, and extracted with ethyl acetate. The resulting ethyl acetate solution was washed with water and dried. Ethyl acetate was evaporated under reduced pressure to give 25.4 gof an oily product. The resulting oily product was crystallized from ethanol/acetone to give 18.4g of pure 1-(4-chlorophenylcarbamoyl)-3-(4-phenoxyphenyl)-4-(4-chlorophenyl)-2-pyrazoline.

Melting point: 158.7 - 160.9 °C.

$\nu_{max}^{KBr}$ ($cm^{-1}$): 3400, 1650, 1585, 1525, 1500, 1490, 1240.

$\delta_{TMS}^{CDCl_3}$ (ppm): 3.94 (1H, dd, J=5Hz, 11Hz), 4.30 (1H, t, J-11Hz), 4.63 (1H, dd, J=5Hz, 11Hz), 6.7-7.6 (18H, m), 8.04 (1H, broad s).

Elemental analysis values: $C_{28}H_{21}Cl_2N_3O_2$

| | C | H | Cl | N |
|---|---|---|---|---|
| Calculated (%) | 66.94 | 4.21 | 14.11 | 8.37 |
| Found (%) | 66.78 | 4.28 | 14.22 | 8.37 |

## SYNTHESIS EXAMPLE 3

Synthesis of 1-(4-fluorophenylcarbamoyl)-3-(4-phenoxyphenyl)-4-(4-fluorophenyl)-2-pyrazoline (compound No. 7):-

In 100 ml of anhydrous tetrahydrofuran was dissolved 18.0g of 3-(4-phenoxyphenyl)-4-(4-fluorophenyl)-2-pyrazoline, and then 7.4g of 4-fluorophenyl isocyanate was added. The mixture was stirred at room temperature for 3 hours. Tetrahydrofuran was evaporated under reduced pressure to give 25.4g of a residue. The residue was crystallized from ethanol to give 23.4g of pure 1-(4-fluorophenylcarbamoyl)-3-(4-phenoxyphenyl)-4-(4-fluorophenyl)-2-pyrazoline.

0207728

Melting point: 122.6 - 123.6 °C.

$\nu_{max}^{KBr}$ ($cm^{-1}$): 3400, 1650, 1535, 1510, 1490, 1425, 1240.

$\delta_{TMS}^{CDCl_3}$ (ppm): 4.02 (1H, dd, J=5Hz, 11Hz), 4.38 (1H, t, J=11Hz), 4.70 (1H, dd, J=5Hz, 11Hz), 6.8-7.7 (17H, m), 8.09 (1H, broad s).

SYNTHESIS EXAMPLE 4

Synthesis of 1-(4-fluorophenylcarbamoyl)-3-(4-phenoxyphenyl)-4-(4-methylsulfonyloxyphenyl)-2-pyrazoline (compound No. 12):-

In 20 ml of methylene chloride was dissolved 1.5g of 3-(4-phenoxyphenyl)-4-(4-methylsulfonyloxyphenyl)-2-pyrazoline, and then 0.50g of 4-fluorophenyl isocyanate was added. The mixture was stirred overnight at room temperature. Methylene chloride was evaporated. The oily residue was purified by column chromatography to give 0.70g of pure 1-(4-fluorophenylcarbamoyl)-3-(4-phenoxyphenyl)-4-(4-methylsulfonyloxyphenyl)-2-pyrazoline.

Melting point: 168 - 171 °C

$\nu_{max}^{KBr}$ ($cm^{-1}$): 3300, 1650, 1600, 1510, 1230, 1150.

$\delta_{TMS}^{CDCl_3}$ (ppm): 3.12 (3H, s), 4.04 (1H, dd, J=5Hz, 11Hz), 4.39 (1H, t, J-11Hz), 4.76 (1H, dd, J=5Hz, 11Hz), 6.82-8.12 (18H, m), 8.08 (1H, broad s).

Elemental analysis values: $C_{29}H_{24}FN_3O_5S$

| | C | H | F | N | S |
|---|---|---|---|---|---|
| Calculated (%) | 63.84 | 4.43 | 3.48 | 7.70 | 5.88 |
| Found (%) | 63.89 | 4.41 | 3.42 | 7.72 | 5.91 |

SYNTHESIS EXAMPLE 5

Synthesis of 1-(4-fluorophenylcarbamoyl)-3-(4-phenoxyphenyl)-4-(4-bromophenyl)-2-pyrazoline (compound No. 14):-

In 10 ml of anhydrous tetrahydrofuran was dissolved 2.0g of 3-(4-phenoxyphenyl)-4-(4-bromophenyl)-2-pyrazoline, and 0.70g of 4-fluorophenyl isocyanate. The mixture was stirred overnight at room temperature. Tetrahydrofuran was evaporated under reduced pressure. The resulting residue was crystallized from ethanol to give 1.8g of 1-(4-fluorophenylcarbamoyl)-3-(4-phenoxyphenyl)-4-(4-bromophenyl)-2-pyrazoline.

Melting point: 146.5 - 147.5 °C

$\nu_{max}^{KBr}$ ($cm^{-1}$): 3420, 3240, 1665, 1595, 1540, 1520, 1435, 1320, 1250, 1015, 835.

$\delta_{TMS}^{CDCl_3}$ (ppm): 3.99 (1H, dd, J=5Hz, 10Hz), 4.34 (1H, t, J=10Hz), 4.65 (1H, dd, J=5Hz, 10Hz), 6.92-7.76 (17H, m), 8.09 (1H, broad s).

Elemental analysis values: $C_{28}H_{21}BrFN_3O_2$

| | C | H | Br | F | N |
|---|---|---|---|---|---|
| Calculated (%) | 63.41 | 3.99 | 15.06 | 3.58 | 7.92 |
| Found (%) | 63.38 | 3.96 | 15.02 | 3.55 | 7.88 |

SYNTHESIS EXAMPLE 6

Synthesis of 1-(4-chlorophenylcarbamoyl)-3-(4-anilinophenyl)-4-phenyl-2-pyrazoline (compound No. 15):-

In 10 ml of 1,3-dimethyl-2-imidazolidinone was dissolved 2.5g of 3-(4-anilinophenyl)-4-phenyl-2-pyrazoline, and then 1.23g of 4-chlorophenyl isocyanate was added. The mixture was stirred at room temperature for 3 hours. The reaction mixture was poured into water

and extracted with toluene. The toluene solution was washed with water, and dried, and toluene was evaporated under reduced pressure. The resulting oily product was purified by column chromatography [silica gel; eluent, hexane/ethyl acetate (4:1)] to give 3.2 g of 1-(4-chlorophenylcarbamoyl)-3-(4-anilinophenyl)-4-phenyl-2-pyrazoline.

Melting point: 197.0 - 199.0 °C

$\nu_{max}^{KBr}$ ($cm^{-1}$): 3420, 3340, 1670, 1600, 1520, 1500.

$\delta_{TMS}^{CDCl_3}$ (ppm): 4.02 (1H, dd, J=5.0, 11.0Hz), 4.35 (1H, t, J=11.0Hz), 4.70 (1H, dd, J=5.0, 11.0Hz), 5.92 (1H, broad s), 6.8-7.7 (18H, m), 8.15 (1H,broad s).

Elemental analysis values: $C_{28}H_{23}ClN_4O$

| | C | H | Cl | N |
|---|---|---|---|---|
| Calculated (%) | 72.02 | 4.96 | 7.59 | 12.00 |
| Found (%) | 71.97 | 4.91 | 7.57 | 11.95 |

SYNTHESIS EXAMPLE 7

Synthesis of 1-(4-fluorophenylcarbamoyl)-3-(4-anilinophenyl)-4-phenyl-2-pyrazoline (compound No. 16):-

In 20 ml of methylene chloride was dissolved 2.5g of 3-(4-anilinophenyl)-4-phenyl-2-pyrazoline, and 1.09g of 4-fluorophenyl isocyanate was added. The mixture was stirred for one day at room temperature, and the solvent was evaporated under reduced pressure. The resulting oily product was purified by column chromatography [silica gel; eluent,hexane/ethyl acetate (4:1)] to give 2.9 g of 1-(4-fluorophenylcarbamoyl)-3-(4-anilinophenyl)-4-phenyl-2-pyrazoline.

Melting point: 188.0-189.3 °C.

$\nu_{max}^{KBr}$ ($cm^{-1}$): 3380, 3300, 1655, 1590, 1510, 1495.

$\delta_{TMS}^{CDCl_3}$ (ppm): 4.03 (1H, dd, J=5.0, 11.0Hz), 4.38 (1H, t, J=11.0Hz), 4.70 (1H, dd, J=5.0, 11.0Hz), 5.90 (1H, broad s), 6.8-7.7 (18H, m), 8.11 (1H,broad s).

Elemental analysis values: $C_{28}H_{23}FN_4O$

| | C | H | F | N |
|---|---|---|---|---|
| Calculated (%) | 74.65 | 5.15 | 4.22 | 12.44 |
| Found (%) | 74.78 | 5.08 | 4.20 | 12.35 |

SYNTHESIS EXAMPLE 8

Synthesis of 1-(4-fluorophenylcarbamoyl)-3-(4-anilinophenyl)-4-(4-fluorophenyl)-2-pyrazoline (compound No. 17):-

In 10 ml of anhydrous tetrahydrofuran was dissolved 1.2g of 3-(4-anilinophenyl)-4-(4-fluorophenyl)-2-pyrazoline, and then 0.50g of 4-fluorophenyl isocyanate was added. The mixture was stirred at room temperature for 2 hours. The solvent was evaporated under reduced pressure. The oily residue was purified by column chromatography, and crystallized from ethanol to give 0.48g of 1-(4-fluorophenylcarbamoyl)-3-(4-anilinophenyl)-4-(4-fluorophenyl)-2-pyrazoline.

Melting point: 145.0 - 146.5 $^{o}C$

$\nu_{max}^{KBr}$ ($cm^{-1}$): 3400, 3320, 1660, 1600, 1520, 1430, 1390, 1320, 1230.

$\delta_{TMS}^{CDCl_3}$ (ppm): 4.00 (1H, dd, J=5Hz, 11Hz), 4.53 (1H, t, J=11Hz), 4.70 (1H, dd, J=5.0, 11Hz), 5.97 (1H, broad s), 6.9-7.6 (17H, m), 8.12 (1H s).

Elemental analysis values: $C_{28}H_{22}F_2N_4O$

| | C | H | F | N |
|---|---|---|---|---|
| Calculated (%) | 71.78 | 4.73 | 8.11 | 11.96 |
| Found (%) | 71.95 | 4.60 | 8.05 | 11.70 |

SYNTHESIS EXAMPLE 9

Synthesis of 1-(4-chlorophenylcarbamoyl)-3-(4-anilinophenyl)-4-(4-chlorophenyl)-2-pyrazoline (compound No. 20):-

In 10 ml of anhydrous tetrahydrofuran was dissolved 1.2 g of 3-(4-anilinophenyl)-4-(4-chlorophenyl)-2-pyrazoline, and then 0.50 g of 4-chlorophenyl isocyanate was added. The mixture was stirred at room temperature for 2 hours. The solvent was evaporated under reduced pressure. The resulting oily residue was purified by column chromatography, and crystallized from ethanol to give 0.50 g of 1-(4-chlorophenylcarbamoyl)-3-(4-anilinophenyl)-4-(4-chlorophenyl)-2-pyrazoline.

Melting point: 145.0 - 146.0 $^{o}C$

$\nu_{max}^{KBr}$ ($cm^{-1}$): 3400, 3320, 1660, 1600, 1430, 1400, 1340, 1240.

$\delta_{TMS}^{CDCl_3}$ (ppm): 4.01 (1H, dd, J=5.0, 11.0Hz), 4.56 (1H, t, J-11.0Hz), 4.71 (1H, dd, J-5.0, 11.0Hz), 6.9-7.8 (18H, m), 8.30 (1H, s).

Elemental analysis values: $C_{28}H_{22}Cl_2N_4O$

| | C | H | Cl | N |
|---|---|---|---|---|
| Calculated (%) | 67.07 | 4.42 | 14.14 | 11.17 |
| Found (%) | 67.21 | 4.41 | 14.08 | 11.09 |

Below are given Synthesis EXamples for the 3-aryl-4-phenyl-2-pyrazoline derivatives of general formula (II) used as the starting material.

SYNTHESIS EXAMPLE 10

Synthesis of 3-(4-phenoxyphenyl)-4-phenyl-2-pyrazoline:-

(1) Diphenyl ether (20.0 g) and 11.0g of anhydrous aluminum chloride were dissolved in 50 ml of carbon disulfide. The solution was stirred at 0 to 5$^{o}$C in a

nitrogen stream, and 12.0g of phenylacetyl chloride was added dropwise to the solution over 30 minutes, and the mixture was stirred at the same temperature for 30 minutes. The reaction mixture was poured into 400 ml of ice water, and extracted with methylene chloride. The extract was washed with water, and dried. Methylene chloride was evaporated, and 100 ml of ethanol was added to the oily residue to crystallize the product. The crystals were separated by filtration, washed with cold ethanol, and dried to give 21.6g of 4-phenoxyphenyl benzyl ketone.

Melting point: 88.9 - 89.8 $^{o}C$.

$\nu_{max}^{KBr}$ ($cm^{-1}$): 1675, 1605, 1590, 1580, 1495.

$\delta_{TMS}^{CDCl_3}$ (ppm): 4.22 (2H, s), 6.9-8.0 (14H, m).

(2) A mixture of 20.0g of the 4-phenoxyphenyl benzyl ketone obtained in (1) above, 60 ml of 37% formalin, 2.5 ml of piperidine, 2.5 ml of acetic acid and 150 ml of methanol was refluxed for 2 hours. The reaction mixture was poured into water, and extracted with methylene chloride. The extract was washed with water, dried, and evaporated under reduced pressure to give 20.3 g of 1-(4-phenoxyphenyl)-2-phenyl-2-propen-1-one as a colorless oily substance.

$\nu_{max}^{neat}$ ($cm^{-1}$): 1670, 1590, 1495, 1250.

$\delta_{TMS}^{CDCl_3}$ (ppm): 5.55 (1H, s), 5.96 (1H, s), 6.9-8.9 (14H, m).

(3) The 1-(4-phenoxyphenyl)-2-phenyl-2-propen-1-one obtained in (2) above (18.9g) was suspended in 70 ml of ethanol, and 20 ml of hydrazine hydrate was added. With

stirring, the mixture was refluxed for 2 hours, and then kept overnight at room temperature. The precipitate was filtered, washed successively with ethanol and hexane, and dried to give 16.4g of 3-(4-phenoxyphenyl)-4-phenyl-2-pyrazoline as colorless crystals.

Melting point: 142.5 - 143.8 $^{o}$C (dec.).

$\nu_{max}^{KBr}$ ($cm^{-1}$): 3320, 1590, 1515, 1495, 1245.

$\delta_{TMS}^{CDCl_3}$ (ppm): 3.50 (1H, dd, J=5Hz, 11Hz), 3.94 (1H, t, J=11Hz), 4.23 (1H, broad s), 4.48 (1H, dd, J=5Hz, 11Hz), 6.8-7.7 (14H, m).

SYNTHESIS EXAMPLE 11

Synthesis of 3-(4-phenoxyphenyl)-4-(4-chlorophenyl)-2-pyrazoline:-

(1) A mixture of 12.0g of diphenyl ether, 11.0g of anhydrous aluminum chloride and 20 ml of anhydrous methylene chloride was stirred over an ice water bath in a nitrogen stream, and 13.3g of 4-chlorophenylacetyl chloride was added dropwise to the mixture over 20 minutes. The mixture was stirred for 1 hour over an ice water bath. The reaction mixture was poured into 100 ml of ice water, and extracted with methylene chloride. The extract was washed with water and dried, and the solvent was evaporated under reduced pressure. The resulting oily product was crystallized from ethanol to give 21.5g of 4-chlorobenzyl 4-phenoxyphenyl ketone.

Melting point: 110.5 - 112.7 $^{o}$C.

$\nu_{max}^{KBr}$ ($cm^{-1}$): 1680, 1580, 1490, 1250, 1230, 1200.

$\delta_{TMS}^{CDCl_3}$ (ppm): 4.20 (2H, s), 6.9-8.0 (13H, m).

(2) A mixture of 23.3 g of 4-chlorobenzyl 4-phenoxyphenyl ketone obtained in (1) above, 60 ml of 37% formalin, 2.5 ml of piperidine, 2.5 ml of acetic acid and 150 ml of methanol was refluxed for 3 hours with stirring. The reaction mixture was kept overnight at room temperarure. The precipitates were filtered, washed with cold methanol, and dried to give 16.0g of 2-(4-chlorophenyl)-1-(4-phenoxyphenyl)-2-propen-1-one.

Melting point: 78.3- 82.7 $^{o}C$.

$\nu_{max}^{KBr}$ ($cm^{-1}$): 1660, 1600, 1580. 1570, 1500, 1490, 1250, 1220, 1195, 1165.

$\delta_{TMS}^{CDCl_3}$ (ppm): 5.63 (1H, s), 6.02 (1H, s), 6.9-7.95 (13H, m).

(3) The 2-(4-chlorophenyl)-1-(4-phenoxyphenyl)-2-propen-1-one obtained in (2) above (15.0g) was suspended in 50 ml of ethanol, and 16 ml of hydrazine hydrate was added. The mixture was refluxed for 3 hours with stirring. The mixture was kept overnight at room temperature. The precipitates were filtered, washed successively with ethanol and hexane, and dried to give 12.2g of 3-(4-phenoxyphenyl)-4-(4-chlorophenyl)-2-pyrazoline.

$\delta_{TMS}^{CDCl_3}$ (ppm): 3.43 (1H, dd, J=5Hz, 11Hz), 3.88 (1H, t, J=11Hz), 4.18 (1H, dd, J=5Hz, 11Hz), 4.6-5.6 (1H), 6.6-7.6 (14H, m).

SYNTHESIS EXAMPLE 12

Synthesis of 3-(4-phenoxyphenyl)-4-(4-fluorophenyl)-2-pyrazoline:-

(1) A mixture of 12.5g of diphenyl ether, 10.1g of anhydrous aluminum chloride and 11.9g of 4-fluoroacetyl

chloride was treated in the same procedure described in Synthesis Example 11-(1) to give 17.3g of 4-fluorobenzyl 4-phenoxyphenyl ketone.

Melting point: 100.5 - 101.0 $^{o}C$

$\nu_{max}^{KBr}$ ($cm^{-1}$): 1675, 1595, 1585, 1515, 1490, 1270, 1230.

$\delta_{TMS}^{CDCl_3}$ (ppm): 4.19 (2H, s), 6.9-8.0 (13H, m).

(2) The 4-fluorobenzyl 4-phenoxyphenyl ketone obtaind in (1) above (15.0g) was treated in the same procedure described in Synthesis Example 11-(2) to give 14.8g of 1-(4-phenoxyphenyl)-2-(4-fluorophenyl)-2-propen-1-one.

Melting point: 59.8 - 60.2 $^{o}C$

$\nu_{max}^{KBr}$ ($cm^{-1}$): 1660, 1600, 1580, 1500, 1490, 1250.

$\delta_{TMS}^{CDCl_3}$ (ppm): 5.56 (1H, s), 5.96 (1H, s), 6.9-7.95 (13H, m).

(3) The 1-(4-phenoxyphenyl)-2-(4-fluorophenyl)-2-propen-1-one obtained in (2) above was treated in the same procedure described in Synthesis Example 11-(3) to give 12.9g of 3-(4-phenoxyphenyl)-4-(4-fluorophenyl)-2-pyrazoline.

$\delta_{TMS}^{CDCl_3}$ (ppm): 3.44 (1H, dd, J=5Hz, 11Hz), 3.87 (1H, t, J=11Hz), 4.41 (1H, dd, J=5Hz, 11Hz), 4.6-5.6 (1H), 6.5-7.6 (13H, m).

SYNTHESIS EXAMPLE 13

Synthesis of 3-(4-phenoxyphenyl)-4-(4-bromophenyl)-2-pyrazoline:-

(1) Diphenyl ether (11.0 g) and 3.4g of anhydrous aluminum chloride were added to 50 ml of methylene chloride, and a solution of 5.0g of 4-bromophenylacetyl chloride in 10 ml of methylene chloride was added dropwise to the mixture at 5 to 10°C over 30 minutes. Furthermore, the mixture was stirred at the above temperature for 1.5 hours. The reaction mixture was poured into ice water, and extracted with methylene chloride. The extract was washed with water, dried, and evaporated under reduced pressure. The residue was purified by column chromatography to give 6.4g of pure 4-phenoxyphenyl 4-bromobenzyl ketone.

Melting point: 129.5- 130.5 °C.

$\nu_{max}^{KBr}$ ($cm^{-1}$): 1690, 1590, 1500, 1420, 1340, 1255, 1210, 1175, 1000.

$\delta_{TMS}^{CDCl_3}$ (ppm): 4.10 (2H, s), 6.9-8.1 (13H, m).

(2) A mixture of 6.0g of the 4-phenoxyphenyl 4-bromobenzyl ketone obtained in (1) above, 20 ml of 37% formalin, 0.4 ml of piperidine, 0.4 ml of acetic acid and 50 ml of methanol was refluxed for 2 hours with stirring. The reaction mixture was poured into ice water, and extracted with toluene. The toluene extract was washed with water and dried, and the solvent was evaporated under reduced pressure. The resulting residue was purified by column chromatography to give 5.1g of 1-(4-phenoxyphenyl)-2-(4-bromophenyl)-2-propen-1-one.

Melting point: 91.5 - 93.0 °C.

$\nu_{max}^{KBr}$ ($cm^{-1}$): 1670, 1595, 1495, 1425, 1260, 1230, 1170, 1075, 1000.

$\delta_{TMS}^{CDCl_3}$ (ppm): 5.56 (1H, s), 5.95 (1H, s), 6.2-8.0 (13H, m).

(3) A mixture of 5.0g of the 1-(4-phenoxyphenyl)-2-(4-bromophenyl)-2-propen-1-one obtained in (2) above, 40 ml of ethanol and 10 ml of hydrazine hydrate was refluxed for 2 hours. The reaction mixture was kept overnight at room temperature. The precipitates were filtered, washed successively with ethanol and hexane, and dried to give 4.0g of 3-(4-phenoxyphenyl)-4-(4-bromophenyl)-2-pyrazoline.

Melting point: 144.5 - 148.0 °C.

$\nu_{max}^{KBr}$ ($cm^{-1}$): 3320, 1595, 1520, 1495, 1250, 1180, 1080, 1015, 840.

$\delta_{TMS}^{CDCl_3}$ (ppm): 3.40 (1H, dd, J-5Hz, 10Hz), 3.83 (1H, t. J=10Hz), 4.38 (1H, dd, J=5Hz, 10Hz), 6.7-7.65 (13H, m).

SYNTHESIS EXAMPLE 14

Synthesis of 3-(4-phenoxyphenyl)-4-(4-methylsulfonyloxyphenyl)-2-pyrazoline:-

(1) Diphenyl ether (12.0g) and 16.7g of 4-methylsulfonyloxyphenylacetyl chloride were treated in the same procedure described in Synthesis Example 11-(1). The resulting residue was purified by column chromatography to give 5.3g of 4-phenoxyphenyl 4-methylsulfonyloxyphenyl ketone.

$\delta_{TMS}^{CDCl_3}$ (ppm): 3.11 (3H, s), 4.23 (2H, s), 6.9-8.1 (13H, m).

(2) The 4-phenoxyphenyl 4-methylsulfonyloxyphenyl ketone obtained in (1) above (4.5g) was treated in the same procedure described in Synthesis Example 11-(2) and subsequently Synthesis Example 11-(3) to give 3.2g of 3-(4-phenoxyphenyl)-4-(4-methylsulfonyloxyphenyl)-2-pyrazoline.

SYNTHESIS EXAMPLE 15

Synthesis of 3-(4-anilinophenyl)-4-(4-fluorophenyl)-2-pyrazoline:-

(1) Bromobenzene (21.0g) and 9.6g of anhydrous aluminum chloride were added to 40 ml of carbon disulfide, and in a nitrogen stream, a solution of 11.2g of 4-fluorophenylacetyl chloride in 40 ml of carbon disulfide was added dropwise to the above mixture at 5 to 10°C. After the addition, the mixture was stirred at room temperature for 2 hours and further at 50°C for 1 hour. The reaction mixture was poured into 200 ml of ice water, and extracted with methylene chloride. The extract was washed with water and dried, and the solvent was evaporated under reduced pressure. The resulting oily product was purified by column chromatography [silica gel; eluent, toluene/hexane (1:1)] to give 8.8g of 4-bromophenyl 4-fluorobenzyl ketone.

$\nu_{max}^{KBr}$ ($cm^{-1}$): 1700, 1590, 1515, 1495, 1410, 1350, 1230.

$\delta_{TMS}^{CCl_4}$ (ppm): 4.21 (2H, s), 6.9-7.4 (4H, m), 7.61 (2H, d, $J_{AB}$=8.5Hz), 7.88 (2H, d, $J_{AB}$=8.5Hz).

(2) A mixture of 7.5g of the 4-bromophenyl 4-fluorobenzyl ketone obtained in (1) above, 4.0g of ethylene glycol, were dissolved in 50 ml of toluene, and 0.1g of p-toluenesulfonic was refluxed for 5 hours, and the water was removed through a Dean-Stark tube. The reaction

mixture was cooled to room temperature, then poured into 200 ml of a saturated aqueous solution of sodium hydrogen carbonate, and extracted with toluene. The extract was washed with water and dried, and the solvent was evaporated under reduced pressure to give 5.6g of 4-bromophenyl 4-fluorobenzyl ketone ethylene acetal as pale yellow crystals.

Melting point: 107.2 - 108.2 °C

$\nu_{max}^{KBr}$ ($cm^{-1}$): 1590, 1500, 1465, 1210, 1000.

$\delta_{TMS}^{CCl_4}$ (ppm): 3.11 (2H, s), 3.6-3.9 (4H, m), 6.9-7.5 (8H, m).

(3) A mixture of 4.3g of the 4-bromophenyl 4-fluorobenzyl ketone ethylene acetal obtained in (2) above, 2.6g of acetanilide, 2.5g of cuprous iodide, 2.6g of potassium carbonate and 15 ml of dimethylacetamide was heated at 150 to 170°C for 10 hours in a nitrogen stream. The reaction mixture was cooled to 80°C, and a solution of 15 g of sodium hydroxide in 15 ml of water was added. The mixture was heated at 80°C for 3 hours with stirring. The reaction mixture was cooled to room temperature and filtered to remove insoluble materials. Water was added to the filtrate, and the mixture was extracted with ethyl acetate. The extract was washed with water and dried, and the solvent was evaporated under reduced pressure. The resulting oily product was purified by column chromatography (Florisil; eluent, toluene) to give 2.92g of 4-anilinophenyl 4-fluorobenzyl ketone ethylene acetal.

$\nu_{max}^{neat}$ ($cm^{-1}$): 3390, 1600, 1515, 1320, 1220, 1040, 1020.

$\delta_{TMS}^{CCl_4}$ (ppm): 3.15 (2H, s), 3.7-3.9 (4H, m), 5.73 (1H, s), 6.9-7.5 (13H, m).

(4) The 4-anilinophenyl 4-fluorobenzyl ketone ethyleneacetal obtained in (3) above (2.92g) was dissolved in 25 ml of acetone, and 7 ml of water and 0.6 ml of methanesulfonic acid were added. The mixture was refluxed for 1.5 hours, and the reaction mixture was extgracted with 100 ml of ethyl acetate. The extract was washed with water and dried, and the solvent was evaporated under reduced pressue to give 2.3g of a crude product. Recrystallizing the crude product from hexane gave 1.75g of 4-anilinophenyl 4-fluorobenzyl ketone.

$\nu_{max}^{KBr}$ ($cm^{-1}$): 3360, 1680, 1605, 1590, 1545, 1440, 1360, 1260.

$\delta_{TMS}^{CCl_4}$ (ppm): 4.20 (2H, s), 6.24 (1H, s), 6.9-7.5 (11H, m), 7.88 (1H, s), 7.97 (1H, s).

(5) A mixture of 1.75 g of the 4-anilinophenyl 4-fluorobvenzyl ketone obtained in (4) above, 7 ml of 37% formalin, 0.4 ml of piperidine, 0.4 ml of acetic acid and 20 ml of methanol was refluxed for 2 hours with stirring. Water (50 ml) was added to the reaction mixture, and the mixture was extracted with methylene chloride. The extract was washed with water and dried, and the solvent was evaporated under reduced pressure to give 2.18g of 1-(4-anilinophenyl)-2-(4-fluorophenyl)-2-propen-1-one as a pale yellow oily substance.

$\nu_{max}^{neat}$ ($cm^{-1}$): 3320, 1640, 1580, 1490, 1320, 1220.

$\delta_{TMS}^{CCl_4}$ (ppm): 5.55 (1H, s), 5.94 (1H, s), 6.30 (1H, s), 6.9-7.5 (11H, m), 7.8-7.9 (2H, m).

(6) The 1-(4-anilinophenyl)-2-(4-fluorophenyl)-2-propen-1-one obtained in (5) above (2.18g) was dissolved in 20 ml of ethanol, and 5.0 ml of hydrazine hydrate was added. The mixture was refluxed for 2.5 hours with stirring. The reaction mixture was kept at room temperature overnight, and then 30 ml of water was added. The mixture was extracted with 100 ml of ethyl acetate. The extract was washed with water and dried, and the solvent was evaporated under reduced pressure to give 2.3g of crude 3-(4-anilinophenyl)-4-(4-fluorophenyl)-2-pyrazoline.

SYNTHESIS EXAMPLE 16

Synthesis of 3-(4-anilinophenyl)-4-(4-chlorophenyl)-2-pyrazoline:-

(1) Anhydrous aluminum chloride (21.0g) was added to 23.0g of bromobenzene, and in a nitrogen stream, 27.0g of 4-chlorophenylacetyl chloride was added dropwise to the mixture at 5 to 10$^{o}$C. After the addition, the mixture was stirred at room tempeature for 5 hours. The reaction mixture was then poured into 500 ml of ice water, and extracted with methylene chloride. The extract was washed with water and dried, and the solvent was evaporated under reduced pressure. The resulting oily product was purified by column chromatography (silica gel; eluent, toluene/hexane) to give 5.6g of 4-bromophenyl 4-chlorobenzyl ketone.

$\nu_{max}^{KBr}$ ($cm^{-1}$): 1700, 1590, 1500, 1350, 1205, 1000.

$\delta_{TMS}^{CCl_4}$ (ppm): 4.23 (2H, s), 7.2-7.4 (4H, m), 7.64 (2H, d, $J_{AB}$=8.8Hz), 7.88 (2H, d, $J_{AB}$=8.8Hz).

(2) A mixture of 5.6g of the 4-bromophenyl 4-chlorobenzyl ketone obtained in (1) above, 2.8g of

ethylene glycol, 40 ml of toluene, and 0.1g of p-toluenesulfonic acid was refluxed for 5 hours. The water was removed through a Dean-Stark tube. The reaction mixture was cooled to room temperature, poured into 200 ml of a saturated aqueous solution of sodium hydrogen carbonate, and extracted with toluene. The extract was washed with water and dried, and the solvent was evaporated under reduced pressure to give 2.94g of 4-bromophenyl 4-chlorobenzyl ketone ethylene acetal as pale yellow crystals.

Melting point: 68.0 - 69.2 °C

$\delta_{TMS}^{CCl_4}$ (ppm): 3.12 (2H, s), 3.6-3.9 (4H, m), 7.0-7.5 (8H, m).

(3) A mixture of 8.4g of the 4-bromophenyl 4-chlorobenzyl ketone ethylene acetal obtained in (2) above, 4.8g of acetanilide, 4.5g of cuprous iodide, 5.0g of potassium carbonate and 30 ml of dimethylacetamide was heated at 150 to 170°C for 12 hours with stirring in a nitrogen stream. The reaction mixture was cooled to 80°C, and a solution of 15g of potassium hydroxide in 15 ml of water was added. The mixture was further heated at 80°C for 3 hours with stirring. The reaction mixture was cooled to room temperature and filtered to remove insoluble materials. Water was added to the filtrate, and the mixture was extracted with ethyl acetate. The extract was washed with water and dried, and the solvent was evaporated under reduced pressure. The resulting oily product was purified by column chromatography (Florisil; eluent, toluene) to give 4.31 g of 4-anilinophenyl 4-chlorobenzyl ketone ethylene acetal.

$\nu_{max}^{neat}$ ($cm^{-1}$): 3440, 1620, 1520, 1340, 1200, 1060, 1040.

$\delta_{TMS}^{CCl_4}$ (ppm): 3.15 (2H, s), 3.7-3.9 (4H, m), 5.76 (1H, s), 6.9-7.3 (13H, m).

(4) The 4-anilinophenyl 4-chlorobenzyl ketone ethyleneacetal obtained in (3) above (3.71g) was dissolved in 30 ml of acetone, and 5 ml of water and 0.6 ml of methanesulfonic acid were added to the solution. The mixture was refluxed for 30 minutes. The reaction mixture was extracted with 100 ml of ethylene acetate. The extract was washed with water and dried, and the solvent was evaporated under reduced pressure to give 2.5g of a crude product. Recrystallizing the crude product from hexane gave 1.95g of 4-anilinophenyl 4-chlorobenzyl ketone as pale yellow crystals.

$\nu_{max}^{KBr}$ ($cm^{-1}$): 3370, 1690, 1595, 1540, 1520, 1440, 1360, 1260.

$\delta_{TMS}^{CCl_4}$ (ppm): 4.24 (2H, s), 6.27 (1H, s), 7.2-7.6 (11H, m), 7.88 (1H, s), 8.00 (1H, s).

(5) A mixture of 1.95g of the 4-anilinophenyl 4-chlorobenzyl ketone obtained in (4) above, 7 ml of 37% formalin, 0.4 ml of piperidine, 0.4 ml of acetic acid and 20 ml of methanol was refluxed for 2 hours with stirring. The reaction mixture was cooled to room temperature, and 50 ml of water was added to the reaction mixture. The mixture was extracted with methylene chloride. The extract was washed with water and dried, and the solvent was evaporated to give 2.36g of 1-(4-anilinophenyl)-2-(4-chlorophenyl)-2-propen-1-one as a pale yellow oily substance.

$\nu_{max}^{neat}$ ($cm^{-1}$): 3320, 1660, 1595, 1500, 1325, 1180.

$\delta_{TMS}^{CCl_4}$ (ppm): 5.55 (1H, s), 5.97 (1H, s), 6.35 (1H, s), 7.0-7.5 (11H, m), 7.8-7.9 (2H, m).

(6) The 1-(4-anilinophenyl)-2-(4-chlorophenyl)-2-propen-1-one obtained in (5) above (2.36g) was dissolved in 20 ml of ethanol, and 5.0 ml of hydrazine hydrate was added. The mixture was refluxed for 2.5 hours with stirring. The reaction mixture was cooled to room temperature, and then 30 ml of water was added. The mixture was extracted with 100 ml of ethyl acetate. The extract was washed with water and dried, and the solvent was evaporated under reduced pressure to give 2.29g of 3-(4-anilinophenyl)-4-(4-chlorophenyl)-2-pyrazoline as a pale yellow oily substance.

SYNTHESIS EXAMPLE 17

Synthesis of 3-(4-anilinophenyl)-4-phenyl-2-pyrazoline:-

(1) Bromobenzene (94.0g) and 53.3g of anhydrous aluminum chloride were added to 80 ml of methylene chloride, and 47.0g of phenylacetyl chloride was added dropwise to the mixture at 5 to 10°C over 1 hour in a nitrogen stream. After the addition, the mixture was stirred at the some temperature for 4 hours. The reaction mixture was poured into ice water, and extracted with methylene chloride. The extract was washed with water and dried, and the solvent was evaporated under reduced pressure. The resulting oily product was purified by column chromatography [silica gel; eluent, toluene/hexane (1:1)] to give 43.0g of 4-bromophenyl benzyl ketone as crystals.

$\nu_{max}^{KBr}$ ($cm^{-1}$): 1683, 1582, 1218, 1200, 992.

(2) A mixture of 13.8g of the 4-bromophenyl benzyl ketone obtained in (1) above, 6.2g of ethylene glycol, 100 ml of toluene, and 0.8g of p-toluenesulfonic acid was refluxed for 8 hours. The water was removed through a Dean-Stark tube. The reaction mixture was cooled to room temperature, poured into a saturated aqueous solution of sodium hydrogen carbonate, and extracted with ethyl acetate. The extract was washed with water and dried, and the solvent was evaporated under reduced pressure. The resulting crude crystals were sludged with hexane to give 7.7g of 4-bromophenyl benzyl ketone ethylene acetal.

Melting point: 72 to 78 °C.

$\nu_{max}^{KBr}$ ($cm^{-1}$): 2890, 1480, 1180, 1030, 1005.

$\delta_{TMS}^{CCl_4}$ (ppm): 3.05 (2H, s), 3.5-3.9 (4H, m), 7.0-7.4 (9H, m).

(3) A mixture of 5.7g of the 4-bromophenyl benzyl ketone ethyleneacetal obtained in (2) above, 4.2g of acetanilide, 3.9g of cuprous iodide, 4.2g of anhydrous potassium carbonate and 50 ml of dimethylacetamide was heated at 170°C for 10 hours with stirring in a nitrogen stream. The reaction mixture was cooled to 80°C, and a solution of 15g of potassium hydroxide in 15 ml of water was added. The mixture was further stirred at 80°C for 30 minutes and then at 130°C for 10 minutes. The reaction mixture was cooled to room temperature, poured into water, and extracted with toluene. The extract was washed with water and dried, and the solvent was evaporated under reduced pressure. The resulting oily product was purifed by column chromatography (Florisil; eluent, toluene) to give 2.7g of 4-anilinophenyl benzyl ketone ethyleneacetal.

Melting point: 93.0 - 94.5 °C.

$\nu_{max}^{KBr}$ ($cm^{-1}$): 3440, 1620, 1520, 1340, 1200, 1060, 1040.

$\delta_{TMS}^{CCl_4}$ (ppm): 3.04 (2H, s), 3.6-3.8 (4H, m), 5.52 (1H, broad s), 6.7-7.3 (14H, m).

(4) The 4-anilinophenyl benzyl ketone ethylene acetal obtained in (3) above (1.8g) was dissolved in 30 ml of acetone, and 15 ml of water and 0.6 ml of methanesulfonic acid were added to the solution. The mixture was refluxed for 1 hour. The reaction mixture was poured into ice water, and extracted with ethyl acetate. The extract was washed with water and dried, and the solvent was evaporated under reduced pressure to give 1.6g of a crude product. The crude product was sludged with hexane-ether to give 1.35g of 4-anilinophenylbenzeyl ketone.

$\nu_{max}^{KBr}$ ($cm^{-1}$): 3350, 1655, 1605, 1585, 1567, 1350.

$\delta_{TMS}^{CDCl_3}$ (ppm): 4.18 (2H, s), 6.17 (1H, broad s), 6.94 (2H, d, $J_{AB}$=8.0Hz), 7.0-7.5 (10H, m), 7.90 (2H, d, $J_{AB}$=8.0Hz).

(5) A mixture of 1.0g of the 4-anilinophenyl benzyl ketone obtained in (4) above, 3 ml of 37% formalin, 0.1 ml of piperidine and 0.1 ml of acetic acid and 20 ml of methanol was refluxed for 4 hours with stirring. The reaction mixture was cooled to room temperature, and 50 ml of water was added. The mixture was extracted with methylene chloride. The extract was washed with water and dried, and the solvent was evaporated under reduced pressure to give 1.05g of 1-(4-anilinophenyl)-2-phenyl-

2-propen-1-one as a pale yellow oily substance.

(6) The 1-(4-anilinophenyl)-2-phenyl-2-propen-1-one obtained in (5) above (1.05g) was dissolved in 20 ml of ethanol. Hydrazine hydrate (1.0 ml) was added to the solution, and the mixture was refluxed for 2.5 hours with stirring. The reaction mixture was cooled to room temperature, and 30 ml of water was added. The mixture was extracted with ethyl acetate. The extract was washed with water and dried, and the solvent was evaporated under reduced pressure to give 1.1g of 3-(4-anilinophenyl)-4-phenyl-2-pyrazoline.

Formulation Examples are shown below for formulating the compounds of this invention represented by general formula (I) as an insecticide. In the following, all parts are by weight.

FORMULATION EXAMPLE 1

Emulsifiable concentrate:-

Ten parts of the compound of the invention, 10 parts of Sorpol 355S (a registered trademark for a product of Toho Chemical Industrial Co., Ltd.) and 80 parts of xylene were mixed and dissolved to form an emuilsifiable concentrate.

FORMULATION EXAMPLE 2

Wettable powder:-

Twenty parts of the compound of this invention, 10 parts of sodium lignosulfonate, 5 parts of sodium alkylnaphthalenesulfonate, 5 parts of white carbon and 60 parts of diatomaceous earth were uniformly mixed and pulverized to form a powder.

FORMULATION EXAMPLE 3

Dust:-

Three parts of the compound of this invention was dissolved in 10 parts of acetone, and 97 parts of clay was added. Acetone was then evaporated to give a dust.

FORMULATION EXAMPLE 4

Granules:-

Three parts of the compound of this invention, 1 part of sodium lignosulfonate, 20 parts of talc and 76 parts of bentonite were mixed and kneaded with a suitable amount of water. The mixture was granulated and dried to give granules.

FORMULATION EXAMPLE 5

Poison bait:-

One part of the compound of this invention, 5 parts of sugar, 50 parts of wheat bran, 20 parts of rice bran, and 24 parts of wheat flour were mixed and kneaded with a suitable amount of water. The mixture was granulated, and dried to give a bait.

The following Test Examples specifically illustrate the superior insecticidal activity of the compounds of this invention. All tests were carried out through three replicates, and the results are expressed by average values.

TEST EXAMPLE 1

Effect againt tobacco cutworm:-

An emulsifiable concentrate prepared as in Formulation Example 1 was diluted to 20,000 times with water, and sweet potato leaves were immersed in the dilution. After air drying, the treated leaves were transferred to plastic cups. Then ten 3rd instar larvae of tobacco cutworm were set free in each cup. After 48 hours, the numbers of killed and living larvae were counted and mortality was calculated. The results are shown in Table 2.

TEST EXAMPLE 2

Effect against house fly:-

Filter paper was placed at the bottom of a glass Petri dish having a diameter of 9 cm and a depth of 2 cm, and a 10,000-fold dilution of an emulsifable concentrate prepared as in Formulation Example 1 was added

dropwise in an amount of 0.5 ml. After the dilution was absorbed uniformly in the filter paper, 10 female imagoes of house flies 3 days after emergence were released into the dish. Absorbent cotton wetted with sugar water was attached to the inside of the dish. Forty-eight hours later, the number of killed imagoes was counted and mortality was calculated. The result are shown in Table 3.

TEST EXAMPLE 3

Effect against resistant green rice leafhopper:-

A dust, prepared as in Formulation Example 3, was sprayed at a rate of 1 kg/10a by a bell jar duster onto 3-leaf stage paddy rice seedlings planted in Wagner pots (1/10,000 a). The treated seedlings were covered with a cylindrical wire gauze, and ten male imagoes of resistant green rice leafhopper (collected at Nakagawara) were released into the cylindrical wire gauze. Forty-eight hours later, the number of killed insects was counted and mortality was calculated. The results are shown in Table 4.

TEST EXAMPLE 4

Effect against diamondback moth:-

A 2,000-fold dilution of an emulsifiable concentrate, prepared as in Formulation Example 1, was sprayed with a hand sprayer onto cabbage seedlings (5- to 6-leaf stage) grown in pots so that the chemical dripped slightly from the seedlings. The treated seedlings were placed in a greenhouse, and immediately after air drying or 10 days after air drying, the leaves were cut off, and put into plastic cups. Then ten 2nd instar larvae of diamondback moth were set free in each cup. After 72 hours, the numbers of killed and living larvae were counted and mortality was calculated. The results are shown in Table 5.

In these tests, a compound of the following

formula (described in GB 1,514,285) was used as a comparative compound.

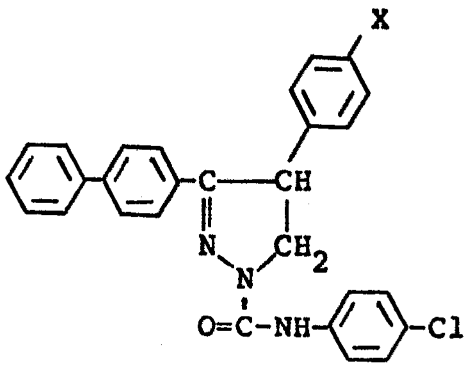

**Table 2**

| Test Compound | Mortality (%) |
|---|---|
| No. 1 | 95 |
| 2 | 100 |
| 3 | 100 |
| 4 | 100 |
| 5 | 100 |
| 6 | 100 |
| 7 | 100 |
| 8 | 90 |
| 9 | 100 |
| 10 | 100 |
| 11 | 100 |
| 12 | 100 |
| 13 | 100 |
| 14 | 100 |
| 15 | 100 |
| 16 | 100 |
| 17 | 100 |
| 18 | 100 |
| 19 | 100 |
| 20 | 90 |
| Comparative compound | 0 |
| Non-treated | 0 |

Table 3

| Test Compound | Mortality (%) |
|---|---|
| No. 1 | 100 |
| 2 | 80 |
| 3 | 100 |
| 4 | 100 |
| 5 | 70 |
| 6 | 100 |
| 7 | 100 |
| 8 | 90 |
| 9 | 75 |
| 11 | 100 |
| 12 | 100 |
| 13 | 100 |
| 16 | 90 |
| 17 | 100 |
| 18 | 100 |
| 19 | 100 |
| Comparative compound | 0 |
| Non-treated | 0 |

Table 4

| Test Compound | Mortality (%) |
|---|---|
| No. 1 | 100 |
| 4 | 100 |
| 6 | 100 |
| 7 | 100 |
| 12 | 100 |
| 15 | 100 |
| 17 | 100 |
| 18 | 100 |
| Comparative compound | 20 |
| Non-treated | 0 |

Table 5

| Test Compound | Mortality (%) | |
|---|---|---|
| | one the day of the treatment | 10 days after the treatment |
| No. 1 | 100 | 100 |
| 3 | 100 | 100 |
| 7 | 100 | 100 |
| 11 | 100 | 100 |
| 12 | 100 | 100 |
| 15 | 100 | 90 |
| 16 | 100 | 100 |
| 17 | 100 | 100 |
| 18 | 100 | 80 |
| 19 | 100 | 100 |
| 20 | 90 | 40 |
| Comparative compound | 13 | 0 |
| Non-treated | 0 | 0 |

It is clear from the foregoing description that the novel pyrazoline compounds of this invention have high insecticidal activity and a broad insecticidal spectrum. These novel pyrazoline compounds can be produced easily by the process of this invention. Agricultural chemicals containing the novel pyrazoline compounds are useful as insecticides having excellent characteristics.

**CLAIMS:**

1. A pyrazoline compound represented by the general formula

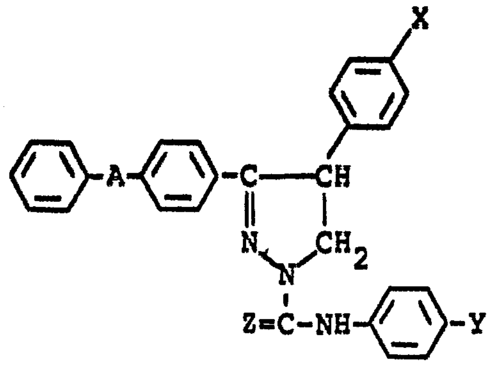

(I)

wherein X represents a hydrogen or halogen atom, or a methyl or methylsulfonyloxy group, Y represents a hydrogen or halogen atom or a trifluoromethyl group, Z represents an oxygen or sulfur atom, and A represents an oxygen atom or -NH-.

2. The pyrazoline compound of claim 1 wherein Z in general formula (I) is an oxygen atom.

3. The pyrazoline compound of claim 1 or claim 2 wherein in general formula (I), X and Y represent a hydrogen or halogen atom, and Z represents an oxygen atom.

4. A process for producing a pyrazoline compound represented by the following formula (I)

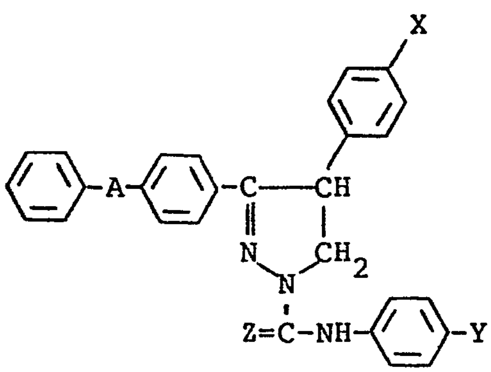

(I)

wherein X represents a hydrogen or halogen atom, or a methyl or methylsulfonyloxy group, Y

represents a hydrogen or halogen atom or a trifluoromethyl group, Z represents an oxygen or sulfur atom, and A represents an oxygen atom or -NH-,

which comprises reacting a compound represented by the general formula

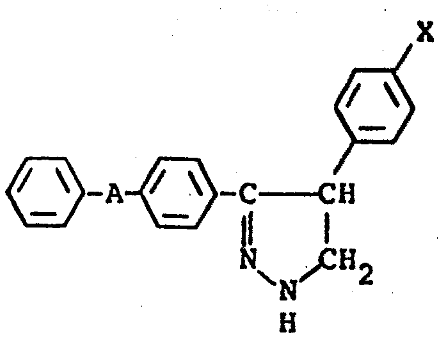

(II)

wherein X and A are as defined above,

with a compound represented by the general formula

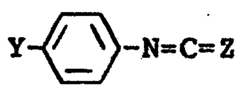

(III)

wherein Y and Z are as defined above.

5. An insecticide comprising as an active ingredient a pyrazoline compound represented by the general formula

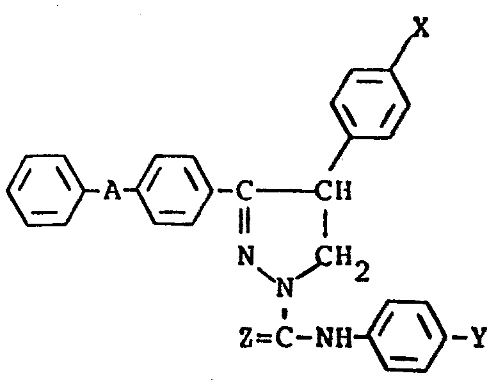

(I)

wherein X represents a hydrogen or halogen atom, or a

methyl or methylsulfonyloxy group, Y represents a hydrogen or halogen atom or a trifluoromethyl group, Z represents an oxygen or sulfur atom, and A represents an oxygen atom or -NH-.

6. An insecticide comprising as an active ingredient a pyrazoline compound according to any of claims 1 to 3 together with one or more carriers and/or adjuvants.